# EUROPÄISCHE PATENTSCHRIFT

(11) **EP 3 253 363 B1**
(45) Veröffentlichungstag und Bekanntmachung des Hinweises auf die Patenterteilung: **26.04.2023**
(21) Anmeldenummer: 16701285.5
(22) Anmeldetag: 21.01.2016
(51) Int. Cl.: A61K 8/34, A61K 8/39, A61K 8/42, A61K 8/46, A61K 8/60, A61Q 19/00, A61K 8/81, A61K 8/86, A61K 8/04

(54) **KÖRPERREINIGUNGS- UND PFLEGEMITTEL**
BODY CLEANSING AND CARE AGENT
PRODUIT DE SOIN ET DE NETTOYAGE DU CORPS

(30) Priorität: 03.02.2015 DE 102015201862
(43) Veröffentlichungstag der Anmeldung: 13.12.2017
(73) Patentinhaber: Henkel AG & Co. KGaA, 40589 Düsseldorf (DE)
(72) Erfinder: SARTINGEN, Kirsten, 41379 Brüggen (DE); HEIDE, Barbara, 47809 Krefeld (DE); PFEIFER, Stephanie, 53773 Hennef (DE)
(86) Internationale Anmeldenummer: PCT/EP2016/051192
(87) Internationale Veröffentlichungsnummer: WO 2016/124407

(56) Entgegenhaltungen:
- EP-A1- 2 786 742
- WO-A1-2015/090744
- WO-A2-2012/110388
- WO-A2-2014/009263
- DE-A1-102011 085 610
- DE-A1-102012 222 956
- US-A1- 2014 121 176
- US-A1- 2014 128 309

## Beschreibung

Die Erfindung liegt auf dem Gebiet der Kosmetik und betrifft Reinigungsmittel, die neben anionischen und amphoteren Tensiden Glycerin und ein spezielles assoziatives Verdickungsmittel enthalten.

Kosmetische Reinigungsmittel enthalten üblicherweise Tenside. Aufgrund ihrer hervorragenden Schaumeigenschaften werden als Primärtenside meist anionische Tenside eingesetzt, die jedoch in höheren Mengen den Nachteil aufweisen können, dass sie die Haut aufweichen und Lipide aus den äußeren Hautschichten entfernen. Dadurch kann die (empfindliche) Haut insbesondere nach häufiger Reinigung mit handelsüblichen Reinigungsmitteln trocken, spröde und mitunter rissig werden, sofern ihr nicht nach oder während der Reinigung Lipide in der Form von Cremes, Lotionen und/oder Pflegeduschbädern wieder zugeführt werden.

In der Vergangenheit wurden zahlreiche Versuche unternommen, mildere kosmetische Reinigungsmittel herzustellen, welche die Haut weniger austrocknen und einen weiteren Behandlungsschritt mit Pflegemitteln entbehrlich werden lassen.

Die mangelnde Stabilität milder Zusammensetzungen, welche oftmals einen hohen Anteil konditionierender Komponenten und/oder einen niedrigen Anteil an (anionischen) Tensiden enthalten, ist jedoch problematisch, denn der Verzicht auf anionische Tenside zugunsten milderer nichtionischer und/oder amphoterer Tenside führt oftmals zu Reinigungsmitteln mit unbefriedigenden Schaumeigenschaften.

Zudem lassen sich viele Pflegewirkstoffe nicht oder nur unter Verwendung zusätzlicher Stabilisierungsmittel in kosmetische Reinigungsmittel einarbeiten.

In der DE102011085610 wurden milde, pflegende kosmetische Reinigungsmittel vorgeschlagen, die äußerst geringe Tensidgehalte aufweisen und dennoch eine reichhaltige Textur und gute Schaumeigenschaften aufweisen.

Bei einigen dieser Produkte führten jedoch bereits geringe Schwankungen in den Zusammensetzungen der eingesetzten Rohstoffe zu Stabilitätsproblemen, so dass ehemals klare Rezepturen während der Lagerung trüb oder neblig wurden und/oder vormals trübe Rezepturen Separationserscheinungen zeigten. In der WO 2014/009263 werden pflegende Reinigungsmittel offenbart, die einen Konditionierungsvorteil bieten.

Der vorliegenden Erfindung lag die Aufgabe zugrunde, milde, schaumstarke, kosmetische Reinigungsmittel zur Verfügung zu stellen, die auch bei höheren Temperaturen hervorragende Stabilitäts- und Lagereigenschaften aufweisen.

Idealerweise sollten schaumstarke, gut hautverträgliche kosmetische Reinigungsmittel hergestellt werden, welche auf der Haut während der Reinigung einen Konditionierungsvorteil hervorrufen.

Es wurde nun überraschend gefunden, dass die zuvor genannten Aufgaben in hervorragendem Maße mithilfe kosmetischer Reinigungsmittel gelöst werden, in denen eine milde Tensidbasis mit Glycerin und speziellen assoziativen Verdickungsmitteln kombiniert wurde.

Die Reinigungsmittel sind lager- und temperaturstabil und erzeugen einen dichten, cremigen Schaum, der durch den Gehalt an speziellen assoziativen Verdickungsmitteln zusätzlich stabilisiert wird.

Darüber hinaus weisen die Reinigungsmittel hervorragende Pflegeeigenschaften auf. Sie hinterlassen nach ihrer Anwendung ein besonders glattes und weiches Hautgefühl und erhalten insbesondere die Feuchtigkeitsbalance der Haut während und nach der Reinigung.

Gegenstand der Erfindung sind daher kosmetische Reinigungsmittel, die in einem wässrigen Träger - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin, und
d) 0,10 bis 3,00 Gew.-% mindestens eines assoziativen Verdickungsmittels enthalten, das ausgewählt ist aus alkoxylierten Estern eines Polyols und mindestens einer C₆-C₃₀-Carbonsäure, wobei die alkoxylierten Ester mindestens eine Struktureinheit -(AO)ₘ-aufweisen, in der AO für eine Ethylenoxidgruppe (EO) und/oder für eine Propylenoxidgruppe (PO) und m für ganze Zahlen zwischen 10 bis 300 steht, und wobei das assoziative Verdickungsmittel ein ethoxylierter Propylenglycol-C8-C24-Carbonsäureester mit mindestens einer Struktureinheit -(EO)n - ist, in der n für ganze Zahlen zwischen 40 und 100 steht,
e) 0,10 bis 3,00 Gew.-% mindestens eines 1-4-fach ethoxylierten C8 -C24 -Alkohols und
f) 0,01 bis 5,00 Gew.-% mindestens eines Alkyl(oligo)glycosids,

- wobei das Gewichtsverhältnis der Tenside a): b) 12 : 1 bis 3,5 : 1 beträgt.

Unter einem wässrigen Träger wird im Rahmen der Erfindung bevorzugt ein wässriger oder wässrigalkoholischer Träger verstanden.

Der wässrige Träger enthält bevorzugt mindestens 55 Gew.-%, mehr bevorzugt mindestens 60 Gew.-%, besonders bevorzugt mindestens 65 Gew.-% und insbesondere bevorzugt mindestens 70 Gew.-% Wasser.

Weiterhin kann der kosmetische Träger 0,01 bis 40 Gew.-%, bevorzugt 0,05 bis 30 Gew.-% und insbesondere 0,1 bis 20 Gew.-% mindestens eines Alkohols enthalten.

Geeignete Alkohole sind beispielsweise Ethanol, Ethyldiglykol, 1-Propanol, 2-Propanol, Isopropanol, 1,2-Propylenglycol, 1,3-Propylenglycol, 1-Butanol, 2-Butanol, 1,2-Butandiol, 1,3-Butandiol, 1,4-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Besonders bevorzugt sind die wasserlöslichen Alkohole.

Insbesondere bevorzugt sind Ethanol, 1,2-Propylenglycol, Benzylalkohol und/oder Phenoxyethanol sowie Mischungen dieser Alkohole.

Unter Estern d) werden bevorzugt alkoxylierte Ester verstanden, bei denen das Polyol ausgewählt ist aus 1,2-Propylenglycol, 1,3-Propylengrycol, 1,2 Butylenglycol, 1,3-Butylengycol, 1,2-Pentandiol, 1,3-Pentandiol, 1,2-Hexandiol, 1,6-Hexandiol, Glycerin, Diglycerin, Polyglycerin, Polyethylenglycol, Mannitol, Isomaltol, Sorbitol, Xylitol, Erythrit, Glucose, Mannose, Fructose, Ribose, Galactose, Fucose, Rhamnose, Saccharose, Lactose, Maltose und/oder Trehalose.

Besonders bevorzugt sind 1,2-Propylenglycol, 1,2 Butylenglycol, 1,2-Hexandiol, 1,6-Hexandiol, Glycerin, Diglycerin, Polyethylenglycol, Mannitol, Sorbitol, Erythrit und/oder Glucose und ganz besonders bevorzugt sind 1,2-Propylenglycol, Glycerin, Sorbitol und/oder Glucose.

Insbesondere bevorzugt enthalten die Ester d) als Polyoleinheit 1,2-Propylenglycol oder Glycerin.

Weiterhin werden unter Estern d) bevorzugt alkoxylierte Ester verstanden, die als C₆-C₃₀-Carbonsäure mindestens eine geradkettige oder verzweigte, gesättigte oder eine einfach oder mehrfach ungesättigte Carbonsäure enthalten. Bevorzugt sind C₈-C₂₄-Carbonsäuren und besonders bevorzugt sind Caprylsäure, Caprinsäure, Laurinsäure, Tridecansäure, Myristinsäure, Palmitinsäure, Stearinsäure, Arachinsäure, Behensäure, Undecylensäure, Myristoleinsäure, Palmitoleinsäure, Petroselinsäure, Ölsäure, Gadoleinsäure, Linolsäure, Linolensäure, Arachidonsäure und/oder Mischungen dieser Säuren.

Erfindungsgemäß besonders bevorzugt sind auch C₈-C₂₄-Carbonsäuremischungen, wie sie erhalten werden, wenn die Carbonsäuren aus natürlichen Fetten und/oder Ölen, wie beispielsweise aus Kokosfett, gewonnen werden.

Ganz besonders bevorzugt sind Caprylsäure, Caprinsäure, Laurinsäure, Tridecansäure, Myristinsäure, Palmitinsäure, Stearinsäure, Behensäure, Ölsäure, Gadoleinsäure, Linolsäure, Linolensäure, Arachidonsäure und/oder Mischungen dieser Säuren, wie sie erhalten werden, wenn sie aus Kokosfett gewonnen werden.

Unter "Estern" d) werden erfindungsgemäß - je nach Hydroxylgruppenanzahl im Polyol - Mono-, Di-, Tri- und/oder Polyester verstanden, die ein- oder mehrfach alkoxyliert sein können.

In der erfindungsgemäßen Ausführungsform sind kosmetische Reinigungsmittel dadurch gekennzeichnet, dass sie als assoziatives Verdickungsmittel d) mindestens einen ethoxylierten Propylenglycol-C₈-C₂₄-Carbonsäureester mit mindestens einer Struktureinheit -(EO)ₙ-enthalten, wobei n für ganze Zahlen zwischen 40 bis 100 steht.

Besonders bevorzugt innerhalb dieser Ausführungsform sind ethoxylierte Mono- und/oder Dipropylenglycolester der Caprylsäure, Caprinsäure, Laurinsäure, Tridecansäure, Myristinsäure, Palmitinsäure, Stearinsäure, Behensäure, Ölsäure und/oder Mischungen dieser Säuren, wie sie erhalten werden, wenn sie aus Kokosfett gewonnen werden, wobei n für ganze Zahlen zwischen 40 bis 100, bevorzugt zwischen 45 und 90 und insbesondere zwischen 50 und 80 steht. Insbesondere bevorzugt innerhalb dieser Ausführungsform ist ein unter der INCI-Bezeichnung PEG-55 Propylene glycol oleate bekanntes und im Handeln von mehreren Anbietern erhältliches assoziatives Verdickungsmittel d).

In einer weiteren offenbarten Ausführungsform sind kosmetische Reinigungsmittel dadurch gekennzeichnet, dass sie als assoziatives Verdickungsmittel d) mindestens einen ethoxylierten Glyceryl-C₈-C₂₄-Carbonsäureester mit mindestens einer Struktureinheit-(EO)ₒ- enthalten, wobei o für ganze Zahlen zwischen 100 bis 250 steht.

Besonders bevorzugt innerhalb dieser Ausführungsform sind ethoxylierte Mono-, Di- und/oder Triglycerinester der Caprylsäure, Caprinsäure, Laurinsäure, Tridecansäure, Myristinsäure, Palmitinsäure, Stearinsäure, Behensäure, Ölsäure und/oder Mischungen dieser Säuren, wie sie erhalten werden, wenn sie aus Kokosfett gewonnen werden, wobei n für ganze Zahlen zwischen 100 und 250, bevorzugt zwischen 120 und 240 und besonders bevorzugt zwischen 140 und 220 steht. Insbesondere bevorzugt innerhalb dieser Ausführungsform ist ein unter der INCI-Bezeichnung PEG-200 Hydrogenated Glyceryl Palmate bekanntes und im Handeln von mehreren Anbietern erhältliches assoziatives Verdickungsmittel d).

In einer weiteren offenbarten Ausführungsform sind kosmetische Reinigungsmittel dadurch gekennzeichnet, dass sie als assoziatives Verdickungsmittel d) mindestens einen ethoxylierten Glucose- oder Sorbitan-C₈-C₂₄-Carbonsäureester mit mindestens einer Struktureinheit -(EO)ₚ- enthalten, wobei p für ganze Zahlen zwischen 10 bis 300, vorzugsweise von 40 bis 250, besonders bevorzugt von 50 bis 200 und insbesondere von 50 bis 150 steht. Insbesondere bevorzugt innerhalb dieser Ausführungsform sind beispielsweise die unter den INCI-Bezeichnungen PEG-120 Methyl glucose dioleate und/oder PEG-80 Sorbitan Laurate bekannten assoziativen Verdickungsmittel d).

Es kann für einige Ausführungsformen der Erfindung weiterhin von besonderem Vorteil sein, als assoziatives Verdickungsmittel d) Mischungen der zuvor genannten alkoxylierten Polyolester einzusetzen. Die Cremigkeit und der Griff des Schaums, den die erfindungsgemäßen Reinigungsmittel bilden, konnte dadurch noch weiter verbessert werden.

In einer weiteren offenbarten Ausführungsform sind daher kosmetische Reinigungsmittel dadurch gekennzeichnet, dass sie
a) mindestens einen ethoxylierten Propylenglycol-C₈-C₂₄-Carbonsäureester mit mindestens einer Struktureinheit -(EO)ₙ- enthalten, wobei n für ganze Zahlen zwischen 40 bis 100 steht, und
b) mindestens einen ethoxylierten Glyceryl-C₈-C₂₄-Carbonsäureester mit mindestens einer Struktureinheit -(EO)ₒ- enthalten, wobei o für ganze Zahlen zwischen 100 bis 250 steht

Die Kombination der unter den INCI-Bezeichnungen PEG-55 Propylene glycol oleate und PEG-200 Hydrogenated Glyceryl Palmate bekannten assoziativen Verdickungsmittel ist innerhalb dieser Ausführungsform besonders bevorzugt.

Die erfindungsgemäßen kosmetischen Reinigungsmittel enthalten das mindestens eine assoziative Verdickungsmittel d) in einem Gewichtsanteil von 0,10 bis 3,00 Gew.-% am Gesamtgewicht des kosmetischen Reinigungsmittels. Bevorzugt ist ein Gewichtsanteil von 0,10 bis 2,50 Gew.-%, mehr bevorzugt von 0,125 bis 2,00 Gew.-%, besonders bevorzugt von 0,15 bis 1,50 Gew.-% und insbesondere von 0,175 bis 1,00 Gew.-%. Die Mengenangaben beziehen sich dabei auf jedes einzelne in dem erfindungsgemäßen Reinigungsmittel enthaltene assoziative Verdickungsmittel d). Zur weiteren Stabilisierung der erfindungsgemäßen kosmetischen Reinigungsmittel - insbesondere zur Stabilisierung der Viskosität und/oder der Rheologie der Zusammensetzungen - kann es weiterhin von Vorteil sein, den kosmetischen Reinigungsmitteln mindestens ein weiteres Verdickungsmittel zuzusetzen. Das weitere Verdickungsmittel sollte mit den in den erfindungsgemäßen Zusammensetzungen enthaltenen Wirkstoffen wie den Tensiden a) und b) sowie mit Glycerin verträglich sein, und die Schaumeigenschaften der Mittel nicht negativ beeinflussen.

Es wurde gefunden, dass alkoxylierte C₆-C₃₀-Alkohole für diesen Zweck besonders geeignet sind.

In einer Ausführungsform enthalten die kosmetischen Reinigungsmittel - bezogen auf ihr Gesamtgewicht - daher zusätzlich 0,10 bis 3,00 Gew.-% mindestens eines alkoxylierten C₆-C₃₀-Alkohols mit einer Struktureinheit -(AO)_{q}-, wobei AO für eine Ethylenoxidgruppe (EO) und/oder für eine Propylenoxidgruppe (PO) und q für ganze Zahlen zwischen 1 bis 4 steht.

Besonders bevorzugt sind 1-4-fach ethoxylierte und/oder propoxylierte, geradkettige oder verzweigte, gesättigte oder eine einfach oder mehrfach ungesättigte C₈-C₂₄-Alkohole, wie beispielsweise Octanol, Decanol, Laurylalkohol, Myristylalkohol, Cetylalkohol, Stearylalkohol, Arachidylalkohol, Behenylalkohol, Oleylalkohol und/oder Elaidylalkohol.

Ganz besonders bevorzugt sind die unter der INCI-Bezeichnung Laureth-(1-4), insbesondere Laureth-2, Myreth-(1-4), insbesondere Myreth-2, Steareth-(1-4), insbesondere Steareth-2 und/oder Oleth-(1-4), insbesondere Oleth-2 bekannten Verbindungen.

Insbesondere bevorzugt ist die unter der INCI-Bezeichnung Laureth-2 bekannte Verbindung.

Es wurde gefunden, dass besonders stabile kosmetische Reinigungsmittel mit hervorragenden Schaum- und Pflegeeigenschaften erhalten werden, wenn diese - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines ethoxylierten Propylenglycol-C₈-C₂₄-Carbonsäureesters mit mindestens einer Struktureinheit -(EO)ₙ-, in der n für ganze Zahlen zwischen 40 bis 100 steht, und
e) 0,10 bis 3,00 Gew.-% mindestens eines 1-4-fach ethoxylierten C₈-C₂₄-Alkohols enthalten.

Die Kombination der Wirkstoffe a) bis e) in den zuvor genannten Mengen ist demnach eine weitere ganz besonders bevorzugte Ausführungsform der offenbarten kosmetischen Reinigungsmittel.

Innerhalb dieser Ausführungsform sind weiterhin kosmetische Reinigungsmittel bevorzugt, die - bezogen auf ihr Gewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines ethoxylierten Propylenglycol-C₈-C₂₄-Carbonsäureesters mit mindestens einer Struktureinheit -(EO)ₙ-, in der n für ganze Zahlen zwischen 40 bis 100 steht,
e) 0,10 bis 3,00 Gew.-% mindestens einen ethoxylierten Glyceryl-C₈-C₂₄-Carbonsäureesters mit mindestens einer Struktureinheit -(EO)ₒ-, in der o für ganze Zahlen zwischen 100 bis 250 steht, und
f) 0,10 bis 3,00 Gew.-% mindestens eines 1-4-fach ethoxylierten C₈-C₂₄-Alkohols enthalten.

Als besonders milde Tensidmischung für die erfindungsgemäßen kosmetischen Reinigungsmittel hat sich eine Mischung aus mindestens einem anionischen Tensid a) und mindestens einem amphoteren/zwitterionischen Tensid b) erwiesen.

Zu den geeigneten anionischen Tensidtypen a), die in den erfindungsgemäßen Reinigungsmitteln eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfate und/oder Alkylethersulfatsalze der Formel R-(OCH₂-CH₂)ₙ-O-SO₃X, in der R bevorzugt eine geradkettige oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 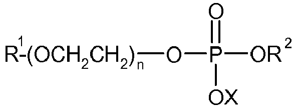 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Bevorzugte anionische Tenside a) sind Alkylsulfate und/oder Alkylethersulfatsalze der Formel R-(OCH₂-CH₂)ₙ-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali-, Erdalkali-, Ammonium- oder Alkanolaminion steht.

Besonders bevorzugte anionische Tenside a) sind geradkettige oder verzweigte Alkylethersulfatsalze der zuvor genannten Formel, die einen Alkylrest R mit 8 bis 18 und insbesondere mit 10 bis 16 C-Atomen sowie 1 bis 6 und insbesondere 2 bis 4 Ethylenoxideinheiten enthalten.

Ganz besonders bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Insbesondere bevorzugt sind die unter der INCI-Bezeichnung Sodium Laureth Sulfate bekannten Tenside.

Der Gewichtsanteil des anionischen Tensids a) am Gesamtgewicht der erfindungsgemäßen Reinigungsmittel beträgt bevorzugt 5,00 bis 15,00 Gew.-%, bevorzugt 6,00 bis 14,00 Gew.-%, besonders bevorzugt 7,00 bis 13,00 Gew.-% und insbesondere 8,00 bis 12,00 Gew.-%.

In einer weiteren bevorzugten Ausführungsform sind erfindungsgemäße kosmetische Reinigungsmittel dadurch gekennzeichnet, dass sie - bezogen auf ihr Gesamtgewicht - als anionisches Tensid a) 5,00 bis 15,00 Gew.-%, bevorzugt 6,00 bis 14,00 Gew.-%, besonders bevorzugt 7,00 bis 13,00 Gew.-% und insbesondere 8,00 bis 12,00 Gew.-% mindestens eines Alkyl(ether)sulfats der allgemeinen Formel R-(OCH₂-CH₂)ₓ-O-SO₃⁻ X⁺ enthalten, in der R eine lineare oder verzweigte, gesättigte oder ungesättigte Alkylgruppe mit 8 bis 30 C-Atomen, x eine der Zahlen 0 oder 1 bis 12 und X⁺ ein Alkalimetall- oder ein Ammoniumion bedeutet, vorzugsweise ein unter der INCI-Bezeichnung Sodium Laureth Sulfate bekanntes anionisches Tensid.

Zu den geeigneten amphoteren und/oder zwitterionischen Tensiden b), die in den erfindungsgemäßen Reinigungsmitteln eingesetzt werden können, zählen beispielsweise eine oder mehrere Verbindungen der nachfolgenden Formeln (I) bis (VII), in denen der Rest R jeweils für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

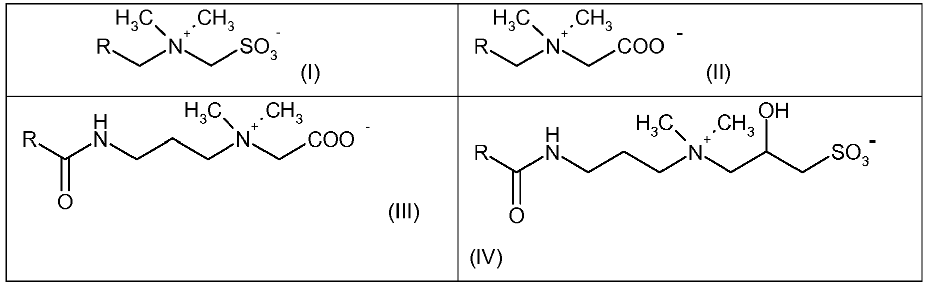

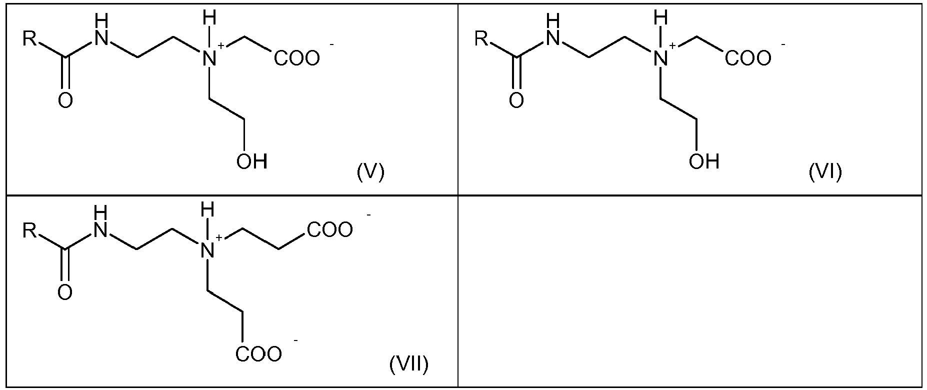

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 8 bis 16 C-Atomen.

Besonders bevorzugt sind amphotere und/oder zwitterionische Tenside, bei denen sich der Rest R von Kokosfett ableitet.

Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren/zwitterionischen Tenside.

Insbesondere bevorzugt sind Tenside mit der INCI-Bezeichnung Cocamidopropylbetain und/oder Lauramidopropylbetain.

Der Gewichtsanteil des mindestens einen amphoteren und/oder zwitterionischen Tensids b) am Gesamtgewicht der erfindungsgemäßen Reinigungsmittel beträgt bevorzugt 0,50 bis 5,00 Gew.-%. Mehr bevorzugt ist ein Gewichtsanteil von 0,60 bis 4,00 Gew.-%, besonders bevorzugt von 0,70 bis 3,50 Gew.-%, ganz insbesondere bevorzugt 0,80 bis 3,00 Gew.-%.

In einer weiteren bevorzugten Ausführungsform sind erfindungsgemäße kosmetische Reinigungsmittel dadurch gekennzeichnet, dass sie - bezogen auf ihr Gesamtgewicht - als amphoteres und/oder zwitterionisches Tensid b) 0,50 bis 5,00 Gew.-%, bevorzugt 0,60 bis 4,00 Gew.-%, besonders bevorzugt 0,70 bis 3,50 Gew.-% und insbesondere 0,80 bis 3,00 Gew.-% mindestens einer Verbindung der nachfolgenden Formel (I) enthalten 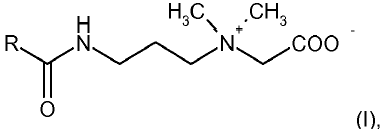 in der R für einen geradkettigen oder verzweigten, gesättigten oder ein- oder mehrfach ungesättigten Alkyl- oder Alkenylrest mit 8 bis 24 Kohlenstoffatomen steht, vorzugsweise ein unter der INCI-Bezeichnung Cocamidopropylbetain bekanntes amphoteres/zwitterionisches Tensid.

Die Milde und die Schaumeigenschaften der erfindungsgemäßen kosmetischen Reinigungsmittel konnten in besonderem Maße durch die sorgfältige Auswahl der Tensidmengen, der Tensidtypen sowie des Gewichtsverhältnisses des mindestens einen anionischen Tensids a) zu dem mindestens einen amphoteren und/oder zwitterionischen Tensid b) gesteigert werden.

Als besonders bevorzugt für die Erzielung einer optimalen Balance zwischen Milde und Schaumeigenschaften der erfindungsgemäßen Mittel hat sich ein Überschuss des mindestens einen anionischen Tensids a) zu dem mindestens einen amphoteren/zwitterionischen Tensid b) und erwiesen.

In der erfindungsgemäßen Ausführungsform beträgt das Gewichtsverhältnis des mindestens einen anionischen Tensids a) zu dem mindestens einen zwitterionischen und/oder amphoteren Tensid b) in den erfindungsgemäßen kosmetischen Reinigungsmitteln 12 : 1 bis 3,5 : 1. Auch offenbart sind Gewichtsverhältnisse von 12 : 1 bis 2 : 1, mehr bevorzugt 11 : 1 bis 2,5 : 1, besonders bevorzugt 10 : 1 bis 3 : 1 und insbesondere 9 : 1 bis 3,5 : 1. Weiterhin bevorzugt ist ein Gesamttensidgehalt von 20 Gew.-%, mehr bevorzugt von 18 Gew.-%, besonders bevorzugt 16 Gew.-% und insbesondere 14 Gew.-%, wobei sich die Mengenangaben auf das Gesamtgewicht des kosmetischen Reinigungsmittels beziehen.

Die Anwesenheit von Glycerin in den erfindungsgemäßen kosmetischen Reinigungsmitteln vermindert die leicht irritative und austrocknende Wirkung der Tenside in den Mitteln und trägt zur Erhöhung der Elastizität und/oder dem Schutz der Haut vor Austrocknung bei.

Damit eine optimale Wirkung erzielt werden kann, ist es von Vorteil, wenn Glycerin in bestimmten Mengenbereichen eingesetzt wird.

Die erfindungsgemäßen kosmetischen Reinigungsmittel enthalten daher Glycerin in einem Gewichtsanteil von 0,10 bis 10,00 Gew.-% am Gesamtgewicht der Mittel. Bevorzugt ist ein Gehalt an Glycerin von 0,30 bis 8,00 Gew.-%, mehr bevorzugt von 0,50 bis 6,00 Gew.-% und insbesondere von 0,75 bis 5,00 Gew.-%.

Es wurde gefunden, dass es zur weiteren Unterstützung der Pflegewirkung der erfindungsgemäßen kosmetischen Reinigungsmittel - insbesondere zur Erhaltung bzw. der Steigerung der Hautelastizität - von Vorteil ist, wenn den Mitteln mindestens ein Vitamin hinzugefügt wird.

Unter geeigneten "Vitaminen" sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   > Vitamin B₁ (Thiamin)
   > Vitamin B₂ (Riboflavin)
   > Vitamin B₃. Unter dieser Bezeichnung werden häufig die Verbindungen Nicotinsäure und Nicotinsäureamid (Niacinamid) geführt.
   > Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   > Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Ganz besonders bevorzugt sind Nicotinsäureamid, Biotin, Pantolacton und/oder Panthenol und insbesondere bevorzugt ist Nicotinsäureamid.

Der Gewichtsanteil des oder der Vitamins(e), Vitaminderivats(e), und/oder der Vitaminvorstufe(n) am Gesamtgewicht der erfindungsgemäßen kosmetischen Reinigungsmittel beträgt bevorzugt 0,005 bis 2,00 Gew.-%, mehr bevorzugt 0,006 bis 1,50 Gew.-%, besonders bevorzugt 0,008 bis 1,00 Gew.-% und insbesondere 0,01 bis 0,50 Gew.-%.

In einer bevorzugten Ausführungsform sind erfindungsgemäße kosmetische Reinigungsmittel dadurch gekennzeichnet, dass sie - bezogen auf ihr Gesamtgewicht - zusätzlich 0,005 bis 2,00 Gew.-%, bevorzugt 0,006 bis 1,50, besonders bevorzugt 0,008 bis 1,00 Gew.-% und insbesondere von 0,01 bis 0,50 Gew.-% mindestens eines Vitamins, eines Vitaminderivats und/oder einer Vitaminvorstufe, vorzugsweise Niacinamid enthalten.

Offenbarte kosmetische Reinigungsmittel enthalten - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines ethoxylierten Propylenglycol-C₈-C₂₄-Carbonsäureesters mit mindestens einer Struktureinheit -(EO)ₙ-, in der n für ganze Zahlen zwischen 40 bis 100 steht,
e) 0,10 bis 3,00 Gew.-% mindestens eines 1-4-fach ethoxylierten C₈-C₂₄-Alkohols und
f) 0,005 bis 2,00 Gew.-% Niacinamid.

Zur weiteren Steigerung der Milde und/oder zur Stabilisierung der Schaumeigenschaften der erfindungsgemäßen Mittel kann es in einer weiteren bevorzugten Ausführungsform von Vorteil sein, wenn die Haarreinigungs- und -pflegemittel - bezogen auf ihr Gewicht - zusätzlich 0,01 bis 5,00 Gew.-%, bevorzugt 0,05 bis 4,00 Gew.-%, besonders bevorzugt 0,10 bis 3,00 Gew.-% und insbesondere 0,25 bis 2,50 Gew.-% mindestens eines nichtionischen Tensids enthalten.

Zu den geeigneten nichtionischen Tensiden zählen beispielsweise
- Aminoxide,
- Fettsäurealkanolamide der nachfolgenden allgemeinen Formel, 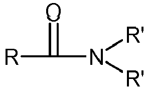 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Anlagerungsprodukte von Ethylenoxid an Fettsäurealkanolamide und Fettamine, und/oder
- Alkyl(oligo)glucoside,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden.

Geeignete Alkyl(oligo)glycoside können bevorzugt ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.

Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.

Besonders bevorzugte Alkyl(oligo)glycoside weisen einen Oligomerisierungsgrad zwischen 1,2 und 1,5 auf.

Der Rest R steht bevorzugt für mindestens einen Alkyl- und/oder Alkenylrest mit 4 bis 24 C-Atomen. Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Geeignete Aminoxide können ausgewählt sein aus mindestens einer Verbindung der allgemeinen Formeln (I) oder (II) 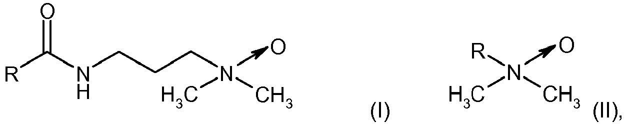 in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.

Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (I) oder (II).

Besonders bevorzugte nichtionische Tenside, die in den erfindungsgemäßen Zusammensetzungen enthalten sein können, sind Alkyl(oligo)glucoside, insbesondere die unter den INCI-Bezeichnungen Caprylyl/Capryl Glucoside, Decyl Glucoside, Lauryl Glucoside und/oder Coco Glucoside bekannten Verbindungen.

In der erfindungsgemäßen Ausführungsform sind erfindungsgemäße kosmetische Reinigungsmittel dadurch gekennzeichnet, dass sie - bezogen auf ihr Gesamtgewicht - zusätzlich 0,01 bis 5,00 Gew.-%, bevorzugt 0,05 bis 4,00 Gew.-%, besonders bevorzugt 0,10 bis 3,00 Gew.-% und insbesondere 0,25 bis 2,50 Gew.-% mindestens eines Alkyl(oligo)glycosids enthalten.

Offenbarte kosmetische Reinigungsmittel enthalten - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines ethoxylierten Propylenglycol-C₈-C₂₄-Carbonsäureesters mit mindestens einer Struktureinheit -(EO)ₙ-, in der n für ganze Zahlen zwischen 40 bis 100 steht,
e) 0,10 bis 3,00 Gew.-% mindestens eines 1-4-fach ethoxylierten C₈-C₂₄-Alkohols und
f) 0,01 bis 5,00 Gew.-% mindestens eines Alkyl(oligo)glycosids.

Neben den zuvor genannten wesentlichen und fakultativen Bestandteilen können die erfindungsgemäßen kosmetischen Reinigungsmittel in einer weiteren bevorzugten Ausführungsform zur weiteren Steigerung der pflegenden Eigenschaften der Mittel mindestens einen weiteren konditionierenden Wirkstoff enthalten, vorzugsweise einen hautkonditionierenden Wirkstoff aus der Gruppe der kationischen Polymere und/oder der natürlichen, synthetischen und/oder mineralischen Öl-, Fett- und/oder Wachskomponenten.

Geeignete kationische Polymere sind beispielsweise:
- quaternisierte Cellulose-Derivate, wie sie unter den Bezeichnungen Celquat^{®} und Polymer JR^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationisierter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte sind kationische Polysaccharidpolymere wie quaternisierte Cellulosepolymere, hydrophob modifizierte kationische Cellulosederivate und/oder kationische Guarderivate sowie polymere Dimethyldiallylammoniumsalze und deren Copolymere mit Estern und Amiden von Acrylsäure und Methacrylsäure. Besonders bevorzugt sind die unter den INCI-Bezeichnungen Guar Hydroxypropyltrimonium Chloride, Polyquaternium-10, Polyquaternium-37, Polyquaternium-67 Polyquaternium-72, Polyquaternium-6 und/oder Polyquaternium-7 bekannten kationischen Polymere; insbesondere bevorzugt ist Polyquaternium-7.

In einer offenbarten Ausführungsform sind kosmetische Reinigungsmittel dadurch gekennzeichnet, dass sie - bezogen auf ihr Gesamtgewicht - zusätzlich 0,01 bis 3,00 Gew.-%, bevorzugt 0,05 bis 2,50 Gew.-%, besonders bevorzugt 0,10 bis 2,00 Gew.-% und insbesondere 0,20 bis 1,50 Gew.-% mindestens eines kationischen Polymeren enthalten, vorzugsweise ein unter der INCI-Bezeichnung Polyquaternium-7 bekanntes kationisches Polymer.

Besonders bevorzugte kosmetische Reinigungsmittel innerhalb dieser Ausführungsform enthalten - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines ethoxylierten Propylenglycol-C₈-C₂₄-Carbonsäureesters mit mindestens einer Struktureinheit -(EO)ₙ-, in der n für ganze Zahlen zwischen 40 bis 100 steht,
e) 0,10 bis 3,00 Gew.-% mindestens eines 1-4-fach ethoxylierten C₈-C₂₄-Alkohols und
f) 0,01 bis 3,00 Gew.-% mindestens eines kationischen Polymeren, vorzugsweise eines unter der INCI-Bezeichnung Polyquaternium-7 bekannten kationischen Polymeren.

Unter geeigneten natürlichen (pflanzlichen) Ölen werden üblicherweise Triglyceride und Mischungen von Triglyceriden verstanden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und/oder Shea-Butter.

Bevorzugt sind Mandelöl, Jojobaöl, Kokosöl und/oder Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol^{®} OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, lsofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12, Cetiol^{®} HE oder Cutina^{®} MD.

Unter geeigneten synthetischen Ölen werden vorzugsweise Silikone, wie beispielsweise
(i) Polyalkylsiloxane, Polyarylsiloxane, Polyalkylarylsiloxane, die flüchtig oder nicht flüchtig, geradkettig, verzweigt oder cyclisch, vernetzt oder nicht vernetzt sein können;
(ii) Polysiloxane, die in ihrer allgemeinen Struktur eine oder mehrere organofunktionelle Gruppen enthalten, die ausgewählt sein können aus:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) lineare Polysiloxan(A)- Polyoxyalkylen(B)- Blockcopoylmere vom Typ (A-B)ₙ mit n > 3;
(iv) gepfropfte Siliconpolymere mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropfte Siliconpolymere mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemische verstanden.

Die natürlichen, synthetischen und/oder mineralischen Öl-, Fett- und/oder Wachskomponenten können in den erfindungsgemäßen kosmetischen Reinigungsmitteln - bezogen auf deren Gesamtgewicht - bevorzugt in einer Menge von 0,005 bis 10,00 Gew.-%, besonders bevorzugt von 0,0075 bis 7,50 Gew.-% und insbesondere bevorzugt von 0,01 bis 5,00 Gew.-% eingesetzt werden. Bevorzugt enthalten die erfindungsgemäßen kosmetischen Reinigungsmittel mindestens ein natürliches Öl - vorzugsweise Mandelöl.

Die erfindungsgemäßen kosmetischen Reinigungsmittel weisen bevorzugt einen pH-Wert um den Neutralpunkt bzw. im leicht aciden Bereich auf, um das Gleichgewicht in der Hautflora nicht bzw. so wenig wie möglich zu beeinflussen.

In einer siebten bevorzugten Ausführungsform weisen die erfindungsgemäßen kosmetischen Reinigungsmittel daher bevorzugt einen pH-Wert im Bereich von 4,0 bis 8,0, mehr bevorzugt von 4,2 bis 7,0, besonders bevorzugt von 4,3 bis 6,0 und insbesondere bevorzugt von 4,5 bis 5, 0 auf.

Erfindungsgemäß weiterhin bevorzugte kosmetische Reinigungsmittel weisen bevorzugt eine Viskosität im Bereich von 4,000 bis 14,000 mPas, mehr bevorzugt von 5,000 bis 13,000 mPas, besonders bevorzugt von 6,000 bis 12,000 mPas, ganz besonders bevorzugt von 6,500 bis 11,000 mPas und insbesondere von 7,000 bis 10,000 mPas auf (gemessen mit einem Haake Rotationsviskosimeter RV 12 bei 20°C, Meßeinrichtung MV, Spindel MV II, 8 UpM). Reinigungszusammensetzungen dieser Viskosität lassen sich bequem und einfach aus einem Behälter auf die Hand oder die Anwendungsoberfläche applizieren, ohne zwischen den Fingern hindurchzulaufen und zu tropfen. Gleichzeitig ist die Viskosität der Zusammensetzung niedrig genug, damit eine zufriedenstellende Verteilung auf der Anwendungsoberfläche unter Zuhilfenahme der Hände gewährleistet ist.

Weitere Wirk-, Hilfs- und Zusatzstoffe, die in den erfindungsgemäßen kosmetischen Reinigungsmitteln eingesetzt werden können, sind beispielsweise:
- UV-Filter,
- Strukturanten wie Maleinsäure und Milchsäure,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Allantoin und/oder Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Pflanzenextrakte,
- Perlglanzmittel wie EGDS oder PEG-3 Distearate,
- Trübungsmittel wie die unter der INCI-Bezeichnung Styrene/Acrylates-Copolymer bekannten Verbindungen,
- Viskositätsregler wie Elektrolytsalze (NaCl).

Die erfindungsgemäßen kosmetischen Reinigungsmittel sind lager- und temperaturstabil, sie weisen hervorragende Rheologie-, Viskositäts- und Schaumeigenschaften auf und eignen sich insbesondere für die Reinigung und gleichzeitige Pflege der Haut.

Mit den erfindungsgemäßen Mitteln behandelte Haut fühlt sich glatt und weich an, die Hautelastizität und/oder die Feuchtigkeitsbalance der Haut während und nach der Reinigung bleiben erhalten.

Ein offenbarter Gegenstand ist die Verwendung der der Wirkstoffkombination aus
a) mindestens einem anionischen Tensid,
b) mindestens einem amphoteren und/oder zwitterionischen Tensid,
c) Glycerin, und
g) mindestens einem assoziativen Verdickungsmittel, ausgewählt aus alkoxylierten Estern eines Polyols und mindestens einer C₆-C₃₀-Carbonsäure, wobei die alkoxylierten Ester mindestens eine Struktureinheit -(AO)ₘ - aufweisen, in der AO für eine Ethylenoxidgruppe (EO) und/oder für eine Propylenoxidgruppe (PO) und m für ganze Zahlen zwischen 10 bis 300 steht,
zur Steigerung
- des Schaumverhaltens und/oder
- der Schaumqualität und/oder
- des Rheologieverhaltens und/oder
- der Lagerstabilität und/oder
- der Stabilität und/oder
- der Pflegeleistung kosmetischer Reinigungsmittel.

Ein Gegenstand der Erfindung ist die Verwendung des erfindungsgemäßen kosmetischen Reinigungsmittels nach einem der Ansprüche 1-8 zur milden Reinigung und Pflege menschlicher Haut, insbesondere zur Steigerung der Hautfeuchtigkeit und/oder zur Verringerung der Hauttrockenheit.

Für bevorzugte Ausführungsformen der erfindungsgemäßen Verwendung gilt mutatis mutandis das zu den erfindungsgemäßen Mitteln Gesagte.

### Beispiele:

Es wurden die folgenden erfindungsgemäßen kosmetischen Reinigungszubereitungen hergestellt (die Mengenangaben beziehen sich auf Gew.-%):

| | **1** | **2** | **3** | **4** | **5** | **6** |
|---|---|---|---|---|---|---|
| **Natriumlaurylethersulfat (2EO), 70% AS** | 13,00 | 13,00 | 12,00 | 13,00 | 13,00 | 13,00 |
| **Cocamidopropylbetain, 37% AS** | 3,00 | 5,00 | 6,00 | 3,00 | 3,00 | 5,00 |
| **Glycerin 99,5%** | 1,00 | 1,00 | 3,00 | 1,00 | 1,00 | 1,00 |
| **Antil 141^{®1}** | 0,35 | 0,35 | 0,40 | 0,20 | 0,15 | 0,30 |
| **Rewoderm LI S 80^{®2}** | | | | 3,00 | 1,00 | |
| **Arlypon F^{®3}** | 0,35 | 0,35 | 0,40 | 0,20 | 0,15 | 0,30 |
| **Mandelöl** | 0,01 | 0,01 | 0,01 | 0,01 | 0,01 | 0,01 |
| **Polyquaternium-7,9% AS** | 1,50 | 1,50 | 1,50 | 1,50 | 1,50 | 1,50 |
| **Coco-Glucoside, 52% AS** | 1,00 | 1,00 | | | 1,00 | 1,00 |
| **PEG-40 Hydrogenated Castor Oil** | 0,25 | 0,25 | 0,25 | 0,25 | 0,25 | 0,25 |
| **CP Styrene Acrylates Copolymer, 40% AS** | 0,75 | 0,75 | 0,75 | 0,75 | 0,75 | 0,75 |
| **Konservierungsmittel** | q.s. | q.s. | q.s. | q.s. | q.s. | q.s. |
| **pH-Stellmittel (bis pH 4,5** - **5,0)** | q.s. | q.s. | q.s. | q.s. | q.s. | q.s. |
| **Parfum** | q.s. | q.s. | q.s. | q.s. | q.s. | q.s. |
| **Wasser** | ad 100 | ad 100 | ad 100 | ad 100 | ad 100 | ad 100 |

Die erfindungsgemäßen kosmetischen Reinigungsmittel der Beispiele 1-6 weisen gegenüber Vergleichsformulierungen (welche kein Glycerin und/oder kein assoziatives Verdickungsmittel und/oder andere Tensidmengen aufweisen) einen verbesserten, cremigeren Schaum auf. Sie verleihen der behandelten Haut ein sanftes und weiches Hautgefühl, das auch > 1 Stunde nach dem Waschen spürbar ist.

Die Stabilität der erfindungsgemäßen Zusammensetzungen - auch bei höheren Temperaturen - ist sehr gut.

Darüber hinaus lassen sich Parfumöle besonders gut in die erfindungsgemäßen Reinigungsmittel einarbeiten.

Ein weiterer Vorteil der erfindungsgemäßen Zusammensetzungen ist, dass sie sehr hohe Schaummengen liefern, wobei dieser Effekt bei den Zusammensetzungen der Beispiele 2, 3 und 6 besonders ausgeprägt ist.

## Patentansprüche

1. Kosmetische Reinigungsmittel, enthaltend in einem wässrigen Träger - bezogen auf ihr Gesamtgewicht -
a) 5,00 bis 15,00 Gew.-% mindestens eines anionischen Tensids,
b) 0,50 bis 5,00 Gew.-% mindestens eines amphoteren und/oder zwitterionischen Tensids,
c) 0,10 bis 10,00 Gew.-% Glycerin,
d) 0,10 bis 3,00 Gew.-% mindestens eines assoziativen Verdickungsmittels, ausgewählt aus alkoxylierten Estern eines Polyols und mindestens einer C₆-C₃₀-Carbonsäure, wobei die alkoxylierten Ester mindestens eine Struktureinheit -(AO)ₘ - aufweisen, in der AO für eine Ethylenoxidgruppe (EO) und/oder für eine Propylenoxidgruppe (PO) und m für ganze Zahlen zwischen 10 bis 300 steht, und wobei das assoziative Verdickungsmittel ein ethoxylierter Propylenglycol-C₈-C₂₄-Carbonsäureester mit mindestens einer Struktureinheit -(EO)ₙ- ist, in der n für ganze Zahlen zwischen 40 und 100 steht,
e) 0,10 bis 3,00 Gew.-% mindestens eines 1-4-fach ethoxylierten C₈-C₂₄-Alkohols und
f) 0,01 bis 5,00 Gew.-% mindestens eines Alkyl(oligo)glycosids,
- wobei das Gewichtsverhältnis der Tenside a) : b) 12 : 1 bis 3,5 : 1 beträgt.

2. Kosmetische Reinigungsmittel nach Anspruch 1, **dadurch gekennzeichnet, dass** als assoziatives Verdickungsmittel d) ein unter der INCI-Bezeichnung PEG-55 Propylene glycol oleate bekanntes Verdickungsmittel eingesetzt wird.

3. Kosmetische Reinigungsmittel nach einem der vorhergehenden Ansprüche, **dadurch gekennzeichnet, dass** sie - bezogen auf ihr Gesamtgewicht - als anionisches Tensid a) 5,00 bis 15,00 Gew.-%, bevorzugt 6,00 bis 14,00 Gew.-%, besonders bevorzugt 7,00 bis 13,00 Gew.-% und insbesondere 8,00 bis 12,00 Gew.-% mindestens eines Alkyl(ether)sulfats der allgemeinen Formel R-(OCH₂-CH₂)ₓ-O-SO₃⁻ X⁺ enthalten, in der R eine lineare oder verzweigte, gesättigte oder ungesättigte Alkylgruppe mit 8 bis 30 C-Atomen, x eine der Zahlen 0 oder 1 bis 12 und X⁺ ein Alkalimetall- oder ein Ammoniumion bedeutet, vorzugsweise ein unter der INCI-Bezeichnung Sodium Laureth Sulfate bekanntes anionisches Tensid.

4. Kosmetische Reinigungsmittel nach einem der vorhergehenden Ansprüche, **dadurch gekennzeichnet, dass** sie - bezogen auf ihr Gesamtgewicht - als amphoteres und/oder zwitterionisches Tensid b) 0,50 bis 5,00 Gew.-%, bevorzugt 0,60 bis 4,00 Gew.-%, besonders bevorzugt 0,70 bis 3,50 Gew.-% und insbesondere 0,80 bis 3,00 Gew.-% mindestens einer Verbindung der nachfolgenden Formel (I) enthalten 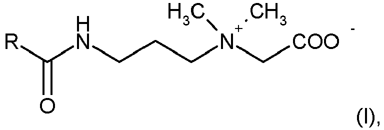 in der R für einen geradkettigen oder verzweigten, gesättigten oder ein- oder mehrfach ungesättigten Alkyl- oder Alkenylrest mit 8 bis 24 Kohlenstoffatomen steht, vorzugsweise ein unter der INCI-Bezeichnung Cocamidopropylbetain bekanntes amphoteres/zwitterionisches Tensid.

5. Kosmetische Reinigungsmittel nach einem der vorhergehenden Ansprüche, **dadurch gekennzeichnet, dass** sie - bezogen auf ihr Gesamtgewicht - zusätzlich 0,005 bis 2,00 Gew.-%, bevorzugt 0,006 bis 1,50, besonders bevorzugt 0,008 bis 1,00 Gew.-% und insbesondere von 0,01 bis 0,50 Gew.-% mindestens eines Vitamins, eines Vitaminderivats und/oder einer Vitaminvorstufe, vorzugsweise Niacinamid enthalten.

6. Kosmetische Reinigungsmittel nach einem der vorhergehenden Ansprüche, **dadurch gekennzeichnet, dass** sie - bezogen auf ihr Gesamtgewicht - zusätzlich 0,01 bis 3,00 Gew.-%, bevorzugt 0,05 bis 2,50 Gew.-%, besonders bevorzugt 0,10 bis 2,00 Gew.-% und insbesondere 0,20 bis 1,50 Gew.-% mindestens eines kationischen Polymeren enthalten, vorzugsweise ein unter der INCI-Bezeichnung Polyquaternium-7 bekanntes kationisches Polymer.

7. Kosmetische Reinigungsmittel nach einem der vorhergehenden Ansprüche, **dadurch gekennzeichnet, dass** sie einen pH-Wert im Bereich von 4,0 bis 8,0, bevorzugt von 4,2 bis 7,0, besonders bevorzugt von 4,3 bis 6,0, außerordentlich bevorzugt von 4,4 bis 5,5 und insbesondere bevorzugt von 4,5 bis 5,0 aufweisen.

8. Kosmetische Reinigungsmittel nach einem der vorhergehenden Ansprüche, **dadurch gekennzeichnet, dass** sie eine Viskosität im Bereich von 4,000 mPas bis 14,000 mPas, bevorzugt von 5,000 mPas bis 13,000 mPas, mehr bevorzugt von 6,000 mPas bis 12,000 mPas, besonders bevorzugt von 6,500 mPas bis 11,000 mPas und insbesondere von 7,000 mPas bis 10,000 mPas aufweisen (gemessen mit einem Haake Rotationsviskosimeter RV 12 bei 20°C, Meßeinrichtung MV, Spindel MV II, 8 UpM).

9. Verwendung eines kosmetischen Reinigungsmittels nach einem der Ansprüche 1 bis 8 zur milden Reinigung und Pflege menschlicher Haut, insbesondere zur Steigerung der Hautfeuchtigkeit und/oder zur Verringerung der Hauttrockenheit.

## Claims

1. Cosmetic cleansing compositions containing, in an aqueous carrier, on a total weight basis
a) 5.00 to 15.00 wt.% of at least one anionic surfactant,
b) 0.50 to 5.00 wt% of at least one amphoteric and/or zwitterionic surfactant,
c) 0.10 to 10.00 wt% glycerol,
d) 0.10 to 3.00 wt.% of at least one associative thickener selected from alkoxylated esters of a polyol and at least one C₆ -C₃₀ -carboxylic acid, the alkoxylated esters having at least one structural unit -(AO)ₘ -, in which AO is an ethylene oxide group (EO) and/or a propylene oxide group (PO) and m is an integer from 10 to 300, and wherein the associative thickener is an ethoxylated propylene glycol C -C₈₂₄ -carboxylic acid ester having at least one structural unit -(EO)ₙ - in which n is an integer from 40 to 100,
e) 0.10 to 3.00% by weight of at least one 1-4-ethylated C₈ -C₂₄ alcohol and
f) 0.01 to 5.00 wt% of at least one alkyl (oligo)glycoside,
- the weight ratio of surfactants a) : b) being from 12 : 1 to 3.5 : 1.

2. The cosmetic cleansing composition according to claim 1, **characterized in that** a thickener known under the INCI designation PEG-55 propylene glycol oleate is used as associative thickener d).

3. Cosmetic cleansing compositions according to one of the preceding claims, **characterized in that** they contain - based on their total weight - as anionic surfactant a) 5.00 to 15.00% by weight, preferably 6.00 to 14.00% by weight, particularly preferably 7.00 to 13.00% by weight and in particular 8.00 to 12.00% of at least one alkyl (ether) sulfate corresponding to the general formula R-(OCH -CH )₂₂ₓ₃⁻ -O-SOX⁺ in which R is a linear or branched, saturated or unsaturated alkyl group containing 8 to 30 carbon atoms, x is one of the numbers 0 or 1 to 12 and X⁺ is an alkali metal ion or an ammonium ion, preferably an anionic surfactant known by the INCI designation sodium laureth sulfate.

4. Cosmetic cleansing compositions according to one of the preceding claims, **characterized in that** they contain - based on their total weight - as amphoteric and/or zwitterionic surfactant b) 0.50 to 5.00% by weight, preferably 0.60 to 4.00% by weight, particularly preferably 0.70 to 3.50% by weight and in particular 0.80 to 3.00% by weight of at least one compound of the following formula (I) 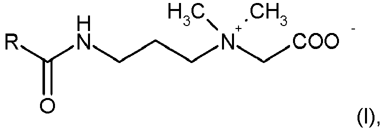 in which R is a straight-chain or branched, saturated or mono- or polyunsaturated alkyl or alkenyl radical containing 8 to 24 carbon atoms, preferably an amphoteric/zwitterionic surfactant known by the INCI name cocamidopropyl betaine.

5. cosmetic cleansing compositions according to any one of the preceding claims, in that **characterized in that** they additionally contain - based on their total weight - from 0.005 to 2.00% by weight, preferably from 0.006 to 1.50% by weight, particularly preferably from 0.008 to 1.00% by weight, and in particular from 0.01 to 0.50% by weight of at least one vitamin, a vitamin derivative and/or a vitamin precursor, preferably niacinamide.

6. Cosmetic cleansing compositions according to one of the preceding claims, **characterized in that** they additionally contain - based on their total weight - from 0.01 to 3.00% by weight, preferably from 0.05 to 2.50% by weight, particularly preferably from 0.10 to 2.00% by weight and especially from 0.20 to 1.50% by weight of at least one cationic polymer, preferably a cationic polymer known under the INCI designation polyquaternium-7.

7. Cosmetic cleansing compositions according to any one of the preceding claims, **characterized in that** they have a pH in the range from 4.0 to 8.0, preferably from 4.2 to 7.0, particularly preferably from 4.3 to 6.0, exceptionally preferably from 4.4 to 5.5 and especially preferably from 4.5 to 5.0.

8. Cosmetic cleansing compositions according to any one of the preceding claims, **characterized in that** they have a viscosity in the range from 4.000 mPas to 14.000 mPas, preferably from 5.000 mPas to 13.000 mPas, more preferably from 6,000 mPas to 12,000 mPas, particularly preferably from 6,500 mPas to 11,000 mPas and especially from 7,000 mPas to 10,000 mPas (measured with a Haake RV 12 rotational viscometer at 20°C, measuring device MV, spindle MV II, 8 rpm).

9. Use of a cosmetic cleansing composition according to any one of claims 1 to 8 for mild cleansing and care of human skin, in particular for increasing skin moisture and/or reducing skin dryness.

## Revendications

1. Compositions cosmétiques nettoyantes contenant dans un véhicule aqueux - par rapport à leur poids total -
a) 5,00 à 15,00 % en poids d'au moins un agent tensioactif anionique,
b) 0,50 à 5,00 % en poids d'au moins un agent tensioactif amphotère et/ou zwitterionique,
c) 0,10 à 10,00 % en poids de glycérol,
d) 0,10 à 3,00 % en poids.% d'au moins un agent épaississant associatif, choisi parmi les esters alcoxylés d'un polyol et d'au moins un acide carboxylique C₆ -C₃₀ , les esters alcoxylés présentant au moins une unité de structure -(AO)ₘ -, dans laquelle AO représente un groupe oxyde d'éthylène (EO) et/ou un groupe oxyde de propylène (PO) et m représente des nombres entiers compris entre 10 et 300, et dans lequel l'épaississant associatif est un ester d'acide propylèneglycol éthoxylé-Cs -C₂₄ - carboxylique ayant au moins une unité de structure -(EO)ₙ-, dans laquelle n représente des nombres entiers compris entre 40 et 100,
e) 0,10 à 3,00 % en poids d'au moins un alcool C₈ -C₂₄ éthoxylé 1 à 4 fois, et
f) 0,01 à 5,00 % en poids d'au moins un alkyl(oligo)glycoside,
- le rapport pondéral des agents tensioactifs a) : b) étant de 12 : 1 à 3,5 : 1.

2. Produits cosmétiques de nettoyage selon la revendication 1, **caractérisés en ce que** l'on utilise comme épaississant associatif d) un épaississant connu sous la désignation INCI PEG-55 Propylene glycol oleate.

3. Produits cosmétiques de nettoyage selon l'une des revendications précédentes, **caractérisés en ce qu'**ils contiennent - par rapport à leur poids total - comme tensioactif anionique a) 5,00 à 15,00 % en poids, de préférence 6,00 à 14,00 % en poids, de manière particulièrement préférée 7,00 à 13,00 % en poids et en particulier 8,00 à 12,00 % en poids de tensioactif anionique.% d'au moins un (éther)sulfate d'alkyle de formule générale R-(OCH -CH )₂₂ₓ₃⁻ - O-SOX⁺ , dans laquelle R représente un groupe alkyle linéaire ou ramifié, saturé ou insaturé, comportant 8 à 30 atomes de carbone, x représente l'un des nombres 0 ou 1 à 12 et X⁺ représente un ion de métal alcalin ou un ion ammonium, de préférence un tensioactif anionique connu sous la désignation INCI Sodium Laureth Sulfate.

4. Produits de nettoyage cosmétiques selon l'une des revendications précédentes, **caractérisés en ce qu'**ils contiennent - par rapport à leur poids total - comme agent tensioactif amphotère et/ou zwitterionique b) 0,50 à 5,00 % en poids, de préférence 0,60 à 4,00 % en poids, de manière particulièrement préférée 0,70 à 3,50 % en poids et en particulier 0,80 à 3,00 % en poids d'au moins un composé de formule (I) suivante 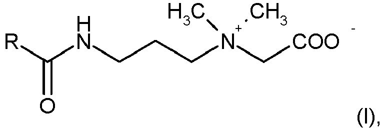 dans laquelle R représente un radical alkyle ou alcényle à chaîne droite ou ramifiée, saturé ou mono- ou polyinsaturé, comportant 8 à 24 atomes de carbone, de préférence un agent tensioactif amphotère/zwitterionique connu sous la désignation INCI cocamidopropylbétaïne.

5. détergents cosmétiques selon l'une des revendications précédentes, en ce que **caractérisés en ce qu'**ils contiennent en outre - par rapport à leur poids total - de 0,005 à 2,00 % en poids, de préférence de 0,006 à 1,50, de manière particulièrement préférée de 0,008 à 1,00 % en poids et en particulier de 0,01 à 0,50 % en poids d'au moins une vitamine, un dérivé de vitamine et/ou un précurseur de vitamine, de préférence le niacinamide.

6. Produits cosmétiques de nettoyage selon l'une des revendications précédentes, **caractérisés en ce qu'**ils contiennent en outre - par rapport à leur poids total - de 0,01 à 3,00 % en poids, de préférence de 0,05 à 2,50 % en poids, de manière particulièrement préférée de 0,10 à 2,00 % en poids et en particulier de 0,20 à 1,50 % en poids d'au moins un polymère cationique, de préférence un polymère cationique connu sous la désignation INCI de polyquaternium-7.

7. Produits cosmétiques de nettoyage selon l'une des revendications précédentes, **caractérisés en ce qu'**ils présentent un pH dans la plage de 4,0 à 8,0, de préférence de 4,2 à 7,0, de manière particulièrement préférée de 4,3 à 6,0, de manière extrêmement préférée de 4,4 à 5,5 et de manière particulièrement préférée de 4,5 à 5,0.

8. Produits cosmétiques nettoyants selon l'une des revendications précédentes, **caractérisés en ce qu'**ils présentent une viscosité comprise entre 4,000 mPas et 14,000 mPas, de préférence entre 5,000 mPas et 13,000 mPas, plus préférentiellement entre 6,000 mPas à 12,000 mPas, de manière particulièrement préférée de 6,500 mPas à 11,000 mPas et en particulier de 7,000 mPas à 10,000 mPas (mesuré avec un viscosimètre rotatif Haake RV 12 à 20°C, dispositif de mesure MV, broche MV II, 8 tr/min).

9. Utilisation d'un produit de nettoyage cosmétique selon l'une des revendications 1 à 8 pour le nettoyage et le soin doux de la peau humaine, en particulier pour augmenter l'hydratation de la peau et/ou pour réduire la sécheresse de la peau.
